(19) Europäisches Patentamt
European Patent Office
Office européen des brevets

(11) Veröffentlichungsnummer: **0 621 287 A1**

# (12) EUROPÄISCHE PATENTANMELDUNG

(21) Anmeldenummer: **94106344.8**

(22) Anmeldetag: **22.04.94**

(51) Int. Cl.5: **C08B 37/16**, A01N 25/10, G01N 33/50

(30) Priorität: **23.04.93 DE 4313408**

(43) Veröffentlichungstag der Anmeldung: **26.10.94 Patentblatt 94/43**

(84) Benannte Vertragsstaaten: **AT BE CH DE DK ES FR GB IT LI NL SE**

(71) Anmelder: **BOEHRINGER MANNHEIM GMBH**
**Sandhofer Strasse 112-132**
**D-68305 Mannheim-Waldhof (DE)**

(72) Erfinder: **Schmidt, Axel, Dr.**
**Gudrunstrasse 18**
**D-80634 München (DE)**
Erfinder: **von der Eltz, Herbert, Dr.**
**In der Au 21**
**D-82362 Weilheim (DE)**
Erfinder: **Kaluza, Klaus, Dr.**
**Hochfeldanger 3**
**D-83670 Bad Heilbrunn (DE)**

(74) Vertreter: **Huber, Bernhard, Dipl.-Chem. et al**
**Patentanwälte**
**H. Weickmann, Dr. K. Fincke**
**F.A. Weickmann, B. Huber**
**Dr. H. Liska, Dr. J. Prechtel, Dr. B. Böhm**
**Postfach 86 08**
**20**
**D-81635 München (DE)**

## (54) Cyclodextrin-Biocid-Komplex.

(57) Die Erfindung betrifft einen Komplex aus Cyclodextrin und einem organischen Biocid, wobei das organische Biocid eine maximale Wasserlöslichkeit von 0,15 % (Gew./Vol.) bei einer Temperatur von 25 °C besitzt, ein Verfahren zur Herstellung des Komplexes sowie die Verwendung des Komplexes zur Konservierung von wäßrigen Lösungen. Die Erfindung betrifft weiterhin eine diagnostische Testlösung, die mindestens einen erfindungsgemäßen Komplex enthält und einen konservierten diagnostischen Testkit, umfassend Testreagenzien und mindestens einen erfindungsgemäßen Komplex.

EP 0 621 287 A1

Die vorliegende Erfindung betrifft neue Komplexe aus Cyclodextrinen und einem organischen Biocid, ein Verfahren zur Herstellung derartiger Komplexe sowie die Verwendung der Komplexe zur Konservierung wäßriger Lösungen, insbesondere diagnostischer Testlösungen. Weiterhin betrifft die vorliegende Erfindung eine diagnostische Testlösung, die mindestens einen neuen Cyclodextrin-Biocid-Komplex enthält.

Biologische, insbesondere diagnostische Testkits enthalten üblicherweise als Reagenzien und Standards Substanzen, die durch Mikroorganismen zersetzbar sind. Zur Verbesserung ihrer Haltbarkeit müssen diese Testkits daher ein Konservierungsmittel enthalten. Bislang hat es sich jedoch als äußerst schwierig erwiesen, geeignete Konservierungsmittel für solche Diagnostika aufzufinden, da neben einer ausreichenden Wirksamkeit gegenüber Bakterien, Hefen und Pilzen die Reaktivität von im Test vorhandenen Substanzen durch das verwendete Konservierungsmittel nicht gestört werden darf. Die Empfindlichkeit von Proteinen, wie z.B. Enzymen oder Antikörpern sowie von Antigenen und Subtraten gegenüber Konservierungsmitteln zeigt sich z.B. darin, daß die Proteine denaturiert werden oder daß immunogene Strukturen zerstört werden können. Darüber hinaus binden zugesetzte Konservierungmittel häufig an Testsubstanzen und stellen auf diese Weise eine Konkurrenz zum eigentlichen Bindepartner im Test dar (z.B. Bindung Enzym/Substrat, Antikörper/Antigen). Bislang war es daher notwendig, für einen spezifischen diagnostischen Test jeweils ein jeweils genau für diesen spezifischen Test geeignetes Konservierungsmittel aufzufinden, welches die zuvor genannten Eigenschaften aufweist. Es ist klar ersichtlich, daß eine derartige Suche nach geeigneten Konservierungsmitteln zeitaufwendig und kostenintensiv ist.

Eine weitere Voraussetzung für den Einsatz von Konservierungsmitteln in wäßrigen Lösungen ist naturgemäß eine ausreichend hohe Wasserlöslichkeit. Bei den meisten gebräuchlichen Biociden handelt es sich um organische Verbindungen. Eine große Anzahl solcher Biocide ist z.B. bei "K.H. Wallhäußer, Praxis der Sterilisation, Desinfektion-Konservierung, Georg Thieme Verlag, Stuttgart, New York" beschrieben.

Ein Nachteil der meisten hochwirksamen organischen Biocide für einen Einsatz in wäßrigen Lösungen, wie etwa diagnostischen Testlösungen, ist jedoch deren oft extrem geringe Wasserlöslichkeit. Nur durch Zusätze wie Detergenzien, organische Lösungsmittel oder durch Herstellung von Emulsionen bzw. Dispersionen lassen sich diese Biocide in wäßrigen Lösungen einsetzen. Diese Zusätze bzw. Konfektionierungen führen jedoch bei Anwesenheit empfindlicher Substanzen wie etwa Proteinen in diagnostischen Tests häufig zu unerwünschten Wechselwirkungen und Ausfällungen, die den Einsatz dieser Biocide unmöglich machen.

Es gibt daher insgesamt nur eine sehr geringe Anzahl an Biociden, die eine genügend große Wasserlöslichkeit für den Einsatz in wäßrigen Lösungen besitzen, so daß für den Einsatz z.B in diagnostischen Tests nur eine begrenzte Anzahl möglicherweise geeigneter Biocide zur Verfügung steht. Selbst diese wenigen Biocide gehen häufig unerwünschte Wechselwirkungen mit Testkomponenten wie z.B. Proteinen ein, so daß der Testentwickler häufig Schwierigkeiten hat, überhaupt ein geeignetes Biocid zu finden. Es besteht deshalb ein großer Bedarf an alternativen wasserlöslichen Biociden.

In der DE-OS 40 22 878.9 wird eine Kombination von mindestens zwei wasserlöslichen Konservierungsmitteln vorgeschlagen, die sich zum Konservieren von diagnostischen Tests eignen. Im Hinblick auf übliche Resistenzentwicklungen von Mikroorganismen ist es jedoch unbedingt erforderlich, auf eine größere Auswahl an geeigneten Biociden zurückgreifen zu können.

Es ist seit längerem bekannt, daß Cyclodextrine bzw. Cyclodextrinderivate mit einer Vielzahl Molekülen Einschlußverbindungen bilden können (siehe z.B. J. Szejtli, Cyclodextrins in Diagnostics, Kontakte (Darmstadt) 1988 (1), 31-36; J. Szejtli, Cyclodextrins in Drug Formulations: Part I und Part II, Pharmaceutical Technology Internation, February 1991, S. 15-23 und March 1991, S. 16-24; W. Saenger, Angew. Chem. 92 (1980), S. 343-361).

Aus der Literatur ist die Verwendung solcher Einschlußverbindungen für die "Mikroverkapselung" von empfindlichen Aromastoffen, Pharmazeutica, Herbiziden und Insektiziden bekannt. Dabei werden für die eingeschlossenen Substanzen häufig vorteilhaftere Eigenschaften erzielt (z.B. Stabilisierung, Änderung der chemischen Reaktivität und günstigere physikochemische Eigenschaften). Auch eine Erhöhung der Löslichkeit bei in Wasser schwer löslichen Verbindungen durch Einschluß in Cyclodextrine ist bekannt.

Auch Einschlußverbindungen von Biociden in Cyclodextrine sind an sich bekannt. So hat der in JP 75-23854 und JP 52-015809 beschriebene Einschlußkomplex von Jod in $\beta$-Cyclodextrin antiseptische und fungizide Eigenschaften.

Aus JP 53-113017 ist beispielsweise die Einlagerung von wasserschwerlöslichen Substanzen wie Sorbinsäure und Benzoesäure zur Haltbarmachung von Nahrungsmitteln über einen weiten pH-Bereich bekannt.

Aus dem Stand der Technik ist jedoch nicht bekannt, in Wasser schwer lösliche Biocide in Cyclodextrine einzulagern, um die Wasserlöslichkeit der Biocide derart zu erhöhen, daß sie direkt zur Konservierung von wäßrigen Systemen und insbesondere von diagnostischen Testlösungen dienen können.

Ein Gegenstand der vorliegenden Erfindung ist somit ein Komplex aus Cyclodextrin und einem organischen Biocid, dadurch gekennzeichnet, daß das organische Biocid eine maximale Wasserlöslichkeit von 0,15 % (Gew./Vol.) bei einer Temperatur von 25 °C besitzt. Vorzugsweise besitzt das organische Biocid eine maximale Wasserlöslichkeit von 0,12 % (Gew./Vol.) und besonders bevorzugt von 0,10 % (Gew./Vol.).

Unter dem Begriff "organisches Biocid" im Sinne der vorliegenden Erfindung sind Substanzen mit mindestens einer Kohlenstoff-Wasserstoff-Bindung zu verstehen. Bevorzugte organische Biocide sind o-Phenylphenol, Densil P, Proxel und Methylenbisthiocyanat, wobei Proxel und Methylenbisthiocyanat am meisten bevorzugt sind. Weitere Beispiele für geeignete organische Biocide sind Hydroxychinolin, Carbendazim oder Dazomet.

Es wurde festgestellt, daß die Komplexe aus Cyclodextrinen und schwer löslichen organischen Biociden hervorragende biocide Eigenschaften aufweisen und praktisch überhaupt keine Wechselwirkung mit Proteinen in wäßriger Lösung eingehen. Dies ist um so überraschender, da nicht-komplexierte Biocide in vielen Fällen mehr oder weniger starke Wechselwirkungen mit Proteinen in wäßrigen Lösungen eingehen, was aber z.B. in diagnostischen Tests sehr unerwünscht ist, da auf diese Weise eine Störung des Tests erfolgt.

Weiterhin wurde überraschenderweise festgestellt, daß die erfindungsgemäßen Komplexe teilweise eine höhere Wirksamkeit als das freie Biocid besitzen. So ist z.B. der Einlagerungskomplex von Methylenbisthiocyanat bezüglich des Wirkstoffgehalts um den Faktor 10 aktiver als der nicht eingelagerte Wirkstoff.

Durch die Einlagerung des Biocids in das Cyclodextrin ist das Biocid komplexiert und es liegt folgendes Gleichgewicht vor:

$$\mathrm{CD} + \mathrm{Biocid} \rightleftharpoons \mathrm{CD} \cdot \mathrm{Biocid}$$

Die Konzentration des freien Biocids ist abhängig von der Dissoziationskonstante $K_D$. Liegt das Gleichgewicht dieser Reaktion zu weit auf der linken Seite, so wäre der Komplex instabil und das in Wasser schlecht lösliche Biocid würde ausfallen. Die erfindungsgemäßen Komplexe zeigen eine so hohe Stabilität, daß eine erhebliche Verbesserung der Löslichkeit des Biocids beobachtet wird.

Die zweite Komponente des erfindungsgemäßen Komplexes ist das Cyclodextrin. Dabei können die unsubstituierten $\alpha$-, $\beta$- und $\Gamma$-Cyclodextrine verwendet werden, aus Gründen der Wasserlöslichkeit der Komplexe ist jedoch die Verwendung derivatisierter Cyclodextrine mit höherer Wasserlöslichkeit bevorzugt. Vorzugsweise werden für diesen Zweck mit $C_1$-$C_4$-Hydroxyalkylgruppen oder/und $C_1$-$C_4$-Alkylgruppen substituierte Cyclodextrine verwendet. Derartige Cyclodextrine sind kommerziell z.B. von der Wacker Chemie GmbH (München, Deutschland) erhältlich. Spezifische Beispiele sind Hydroxypropyl-, Hydroxyethyl- oder Methyl-substituierte Cyclodextrine wie etwa Hydroxypropyl-$\Gamma$-cyclodextrin (MS = 0,6), Hydroxypropyl-$\alpha$-cyclodextrin (MS = 0,6), Hydroxypropyl-$\beta$-cyclodextrin (MS = 0,6 oder 0,9), Hydroxyethyl-$\beta$-cyclodextrin (MS = 0,6 oder 1,0) oder Methyl-$\beta$-cyclodextrin (DS = 1,8). Ein Wert von 0,6 für die molare Substitution (MS) bedeutet dabei, daß durchschnittlich 60 % aller Zuckerreste im Cyclodextrin mit einem entsprechenden Substituenten versehen sind. Ein Substitutionsgrad (DS) von 1,8 bedeutet, daß durchschnittlich 1,8 Substituenten (z.B. Methylgruppen) an einem einzigen Zuckerrest vorliegen.

Die Herstellung der neuen Komplexe kann beispielsweise in wäßriger Lösung des jeweiligen Cyclodextrins auf an sich bekannte Weise erfolgen, indem man das zur Einlagerung zu bringende Biocid zu einer wäßrigen Lösung des jeweiligen Cyclodextrins zugibt und längere Zeit erhitzt. Die unlösliche biocide Substanz geht dabei langsam in Lösung, wobei offenbar der geringe jeweils gelöste Anteil sofort in den Komplex eingelagert wird.

Weiterhin kann die Herstellung der erfindungsgemäßen Komplexe in einem organischen Lösungsmittel erfolgen, wobei nach Bildung des Komplexes das Lösungsmittel z.B. durch Abdampfen wieder entfernt wird. Schließlich ist die Herstellung der erfindungsgemäßen Komplexe auch durch intensives Vormengen zwischen den beiden Komponenten unter Zusatz von wenig Wasser möglich. Grundsätzlich ist in diesem Zusammenhang festzustellen, daß die erfindungsgemäßen Einschlußverbindungen nach bereits beschriebenen Methoden zur Herstellung von Cyclodextrin-Komplexen hergestellt werden können. Ein Gegenstand der vorliegenden Erfindung ist daher auch ein Verfahren zur Herstellung der Komplexe, dadurch gekennzeichnet, daß man geeignete Mengen eines Cyclodextrins und eines organischen Biocids in einem wäßrigen oder organischen Lösungsmittelsystem oder durch intensives Vermengen zusammengibt und den entstehenden Komplex aus dem Reaktionsge-misch gewinnt.

Ein weiterer Gegenstand der vorliegenden Erfindung ist die Verwendung der neuen Komplexe zur Konservierung von wäßrigen Lösungen, wobei die wäßrige Lösung vorzugsweise Proteine enthält. Insbesondere ist es bevorzugt, wenn die Lösung enzymatisch aktive Proteine und/oder immunologisch aktive

Proteine (z.B. Antikörper oder Antikörperfragmente) enthält, die üblicherweise in einer diagnostischen Testlösung vorkommen. Der Begriff "diagnostische Testlösung" bedeutet, daß die Lösung zur Bestimmung eines Analyten mittels enzymatischer, immunologischer oder anderer (z.B. Affinitätsbindung) Methoden vorgesehen ist. Vorzugsweise ist die diagnostische Testlösung zur Bestimmung eines biologischen Analyten in einer Körperflüssigkeit (z.B. Blut, Plasma oder Urin) vorgesehen.

Noch ein weiterer Gegenstand der vorliegenden Erfindung ist eine diagnostische Testlösung, welche dadurch gekennzeichnet ist, daß sie einen erfindungsgemäßen Komplex enthält. Die Konzentration des Komplexes in der Testlösung hängt dabei vom jeweils verwendeten Biocid-Cyclodextrin-Komplex und der jeweils beabsichtigten Anwendung ab. Im allgemeinen hat es sich als zweckmäßig erwiesen, die Komplexe in Konzentrationen von 0,1 µg/ml bis 50 mg/ml, vorzugsweise 1 µg/ml bis 10 mg/ml einzusetzen.

Die Erfindung betrifft schließlich noch einen konservierten diagnostischen Testkit, umfassend Testreagenzien und mindestens eine erfindungsgemäßen Biocid-Cyclodextrin-Komplex. Der erfindungsgemäße Komplex kann im Testkit sowohl dem Puffer als auch dem jeweiligen Testreagens zugesetzt werden. Es hat sich jedoch auch als zweckmäßig erwiesen, den Komplex den Testreagenzien und den Pufferlösungen gleichzeitig zuzusetzen. Bei den Testreagenzien handelt es sich üblicherweise um biologisch aktive Proteine und insbesondere um Enzyme oder/und Antikörper.

Der erfindungsgemäße Komplex hat sich für solche Tests als besonders geeignet erwiesen, die Proteine, insbesondere enzymatisch aktive Proteine, Antikörper oder/und Proteinhormone, Substrate für Enzyme, Hormone, insbesondere T3, T4 bzw. Steroide oder/und Antigene bzw. Haptene enthalten.

Die Erfindung soll weiterhin durch die folgenden Beispiele verdeutlicht werden.

**Beispiel 1**

Herstellung der Einlagerungskomplexe

Als Cyclodextrine wurden die kommerziell erhältlichen Cyclodextrine (Wacker GmbH, München, Deutschland) Alpha W 6 HP 0,9 (Hydroxypropyl-$\alpha$-Cyclodextrin, MS = 0,9), Beta W 7 M 1,8 (Methyl-$\beta$-Cyclodextrin, DS = 1,8) und Gamma W 8 HP 0,6 (Hydroxypropyl-$\gamma$-Cyclodextrin, MS = 0,6) verwendet.

Als Biocide wurden Verbindungen mit einer guten Biocidwirkung und einer geringen Wasserlöslichkeit ausgewählt. Im einzelnen wurden Einlagerungskomplexe von o-Phenylphenol, Methylenbisthiocyanat, Densil P und Proxel hergestellt. Die jeweiligen Biocide sind alle kommerziell erhältlich. Proxel und Densil P, die nur als wäßrige Dispersion im Handel sind, wurden für die Komplexierung vorher als Reinsubstanz isoliert. Die Komplexe wurden dann durch Erhitzen der beiden Komponenten im Verhältnis von etwa 1 Gew.-Teil Biocid zu 9 Gew.-Teilen Cyclodextrin in Wasser und anschließender destillativer Entfernung des Wassers hergestellt.

**Beispiel 2**

Ermittlung der Biocid-Wirkung der erfindungsgemäßen Komplexe

Das Wirkungsspektrum der einzelnen Biocid-Einlagerungskomplexe wurde auf Bakterien (gram-positive und gram-negative Stämme), Hefen und Pilze ermittelt (siehe Tabelle 1). Die Werte sind dabei als MHK-Wert (= minimale Hemmkonzentration) in mg/ml angegeben. Bei einem Vergleich der Werte in Tabelle 1 ist darauf zu achten, daß der Anteil des Wirkstoffs in den Komplexverbindungen < 10 % beträgt, so daß die MHK-Werte für die Komplexe zum direkten Vergleich mit den MHK-Werten der freien Biocide um ca. den Faktor 0,1 multipliziert werden müssen.

Die Bestimmung des Wachstums der Mikroorganismen erfolgte bei Hefen und Pilzen auf M121-Medium (Sabouraud, 2 % Maltose-Nähragar (Merck)) und bei Bakterien auf M130-Medium (Standard I-Nähragar (Merck)) nach 72 Stunden bei 28 °C. Der Anteil des Wirkstoffes in den eingelagerten CD-Verbindungen beträgt < 10 %.

Tabelle 1

| MHK von eingelagerten und freien Biociden (mg/ml) | | | | | | |
|---|---|---|---|---|---|---|
| Biocid | Organismus | | | | | |
| | 1 | 2 | 3 | 4 | 5 | 6 |
| o-Phenylphenol | 1,25 | 1,25 | 1,25 | 1,25 | 0,3 | 0,6 |
| $\gamma$-CD-HP 0,6 o-Phenylphenol | 5,0 | 5,0 | 10,0 | 5,0 | 1,25 | 2,5 |
| Methylenbisthiocyanat | 0,075 | 0,075 | <0,04 | <0,04 | 0,02 | 0,02 |
| $\beta$-CD-Methyl-Methylenbisthiocyanat | 0,075 | 0,15 | <0,04 | 0,15 | 0,04 | 0,04 |
| Densil P | <0,04 | <0,04 | 0,3 | 0,075 | <0,04 | 0,6 |
| $\beta$-CD-Methyl-Densil P | 0,075 | 0,3 | 0,6 | 0,3 | 0,15 | 0,3 |
| Proxel | <0,04 | <0,04 | <0,04 | <0,04 | 0,075 | 0,04 |
| $\alpha$-CD-HP-0,9 Proxel | 0,3 | 1,25 | 0,6 | 0,6 | 1,25 | 0,6 |
| Organismen:<br>1 = Bacillus subtilis (gram +)<br>2 = Staphylococcus aureus (gram +)<br>3 = Pseudomonas fluorescens (gram -)<br>4 = Escherichia coli (gram -)<br>5 = Aspergillus oryzae (Pilz)<br>6 = Candida albicans (Hefe) | | | | | | |

Aus Tabelle 1 geht hervor, daß die Einlagerungskomplexe von o-Phenylphenol, Densil P, insbesondere von Methylenbisthiocyanat und teilweise auch von Proxel bezüglich des Wirkstoffgehalts (unter Berücksichtigung des Wirkstoffanteils in den Komplexen von ca. 10 %) wesentlich aktiver als die freien Biocide sind.

Aus Tabelle 2 ist eine weitere MHK-Wertbestimmung von Biocid-Cyclodextrin-Komplexen an vier unterschiedlichen Keimmischungen (Hefen, Pilze, Bakterien 1, Bakterien 2) ersichtlich. Die Testdurchführung erfolgte wie in Beispiel 1, wobei eine Titerabnahme der Mikroorganismen von zwei 10er Potenzen innerhalb 14 Tagen und eine weitere bis zum Versuchsende nach 6 Wochen bei den Bakterien und eine Titerabnahme von zwei 10er Potenzen innerhalb des gesamten Versuchsverlaufs (6 Wochen) bei Hefen und Pilzen gefordert wurde.

Auch hier beträgt der Anteil des Biocids in den Cyclodextrin-Einlagerungskomplexenca. 10 %.

Tabelle 2

| MHK-Wert Bestimmung eingelagerter Biocide | | | | |
|---|---|---|---|---|
| Biocid | MHK-Wert (mg/ml) | | | |
| | Hefen | Pilze | Bakt.1 | Bakt.2 |
| $\alpha$-CD-HP0,9-Carbendazim | 25 | 50 | 12,5 | 6 |
| $\alpha$-CD-HP0,9-Dazomet | 12,5 | 25 | 25 | > 50 |
| $\alpha$-CD-HP0,9-Proxel | 1,5 | 1,5 | 1,5 | 1,5 |
| $\beta$-CD-Methyl-Carbendazim | 25 | 50 | 12,5 | 6 |
| $\beta$-CD-Methyl-Densil P | 0,2 | 2 | 0,6 | 1 |
| $\beta$-CD-Methyl-Hydroxychinolin | 6 | 6 | >50 | 50 |
| $\beta$-CD-Methyl-Methylenbisthiocyanat | 0,2 | 0,02 | > 0,2 | > 2,5 |
| $\beta$-CD-Methyl-Proxel | 1,5 | 1,5 | 3 | 3 |
| $\gamma$-CD-HP0,6-Carbendazim | 12,5 | 12,5 | 6 | 6 |
| $\gamma$-CD-HP0,6-Dazomet | 3 | 6 | 12,5 | 25 |
| $\gamma$-CD-HP0,6-Hydroxychinolin | 3 | 6 | 25 | 50 |
| $\gamma$-CD-HP0,6-o-Phenylphenol | 6 | 6 | 10 | 10 |

Aus dieser Tabelle ist ersichtlich, daß die erfindungsgemäßen Komplexe nicht nur bei den oben genannten Biociden, sondern auch bei anderen Biociden (wie z.B. Hydroxychinolin, Carbendazim, Dazomet)

eine Wirksamkeit gegen Mikroorganismen zeigen. Die Keimmischungen für den Konservierungstest waren wie folgt:

a) Bakterien 1:
Bacillus subtilis, Escherichia coli, Pseudomonas aeruginosa, Staphylococcus aureus und Enterococcus faecalis.

b) Bakterien 2:
Aeromonas spec., Alcaligenes spec., Flavobacter spec., Proteus vulgaris, Pseudomonas aeruginosa, Pseudomonas fluorescens, Pseudomonas putida.

c) Hefen:
Candida albicans, Rhodotorula rubra, Penicillium glabrum.

d) Pilze:
Aspergillus oryzae, Mucor racemosus.

**Beispiel 3**

Wechselwirkung mit Proteinen

Da z.B. in diagnostischen Tests häufig Enzyme und andere Proteine eingesetzt werden, ist es wünschenswert, daß der Biocid-Cyclodextrin-Komplex keine Wechselwirkung mit dem Protein zeigt. Die Wechselwirkung mit Proteinen wurde durch Ermittlung der Hemmkonzentration (mg/ml) des Biocids auf Enzymtests (Lactatdehydrogenase (LDH)) bzw. auf immunologische Tests (Albumin im Urin (MAU)) bestimmt (siehe Tabelle 3).

Tabelle 3

| Biocid | Biocidmenge pro ml Ansatz mg/ml | Restaktivität LDH*) | MAU**) |
|---|---|---|---|
| α-CD-$HP_{0,9}$-Proxel | 1,5 | 104 % | |
| | 3,0 | 104 % | |
| | 6,0 | 100 % | 94 % |
| ß-CD-Methyl-Densil P | 0,05 | 108 % | |
| | 0,2 | 108 % | |
| | 0,4 | 112 % | |
| | 1,0 | 112 % | 93 % |
| | 2,0 | | 103 % |
| ß-CD-Methyl-Proxel | 0,7 | 110 % | |
| | 1,5 | 110 % | |
| | 3,0 | 110 % | |
| | 6,0 | 105 % | 94 % |
| ß-CD-Methyl-Methylenbisthiocyanat | 1,0 | | 103 % |
| | 2,0 | | 99 % |
| | 2,5 | 104 % | |
| γ-CD-$HP_{0,6}$-o-Phenylphenol | 3,0 | 104 % | |
| | 6,0 | 122 % | |
| | 10,0 | 100 % | 94 % |

*) LDH

Der Test wurde in Halbmikroküvetten, bei T = 25°C (θ = 366nm) durchgeführt.

Testansatz: 0,8 ml 100 mmol/l Tris HCl, pH 7,0
0,1 ml 50 mmol/l Pyruvat
0,1 ml 5 mmol/l NADH

Dazu wurden 10 ng LDH (Schwein, Boehringer Mannheim GmbH, Id.-Nr. 003565) zugegeben.

Der 100 %-Wert wurde ohne Biocidzusatz bestimmt, dann wurde Biocid in den jeweils angegebenen Konzentrationen im Testpuffer gelöst und die Enzymaktivität bestimmt.

**) Tina quant Albumin im Urin (MAU)

Der Test wurde gemäß den Angaben auf dem Beipackzettel des käuflichen Tests (Boehringer Mannheim, Best.-Nr. 1 203 622) durchgeführt. Als 100 %-Wert wurde die Enzymaktivität ohne Biocidzusatz bestimmt. Dann wurde Biocid in den jeweils angegebenen Konzentrationen im Testpuffer gelöst und die Enzymaktivität bestimmt.

## Patentansprüche

1. Komplex aus Cyclodextrin und einem organischen Biocid, **dadurch gekennzeichnet,** daß das organische Biocid eine maximale Wasserlöslichkeit von 0,15 % (Gew./Vol.) bei einer Temperatur von 25 °C besitzt.

2. Komplex nach Anspruch 1, **dadurch gekennzeichnet,** daß das organische Biocid aus der Gruppe ausgewählt ist, die aus o-Phenylphenol, Densil P, Proxel und Methylenbisthiocyanat besteht.

3. Komplex nach Anspruch 2, **dadurch gekennzeichnet,** daß das organische Biocid Methylenbisthiocyanat ist.

4. Komplex nach einem der Ansprüche 1 bis 3, **dadurch gekennzeichnet,** daß das Cyclodextrin ein derivatisiertes Cyclodextrin ist.

5. Komplex nach Anspruch 4, **dadurch gekennzeichnet,** daß das Cyclodextrin durch $C_1$-$C_4$-Hydroxylalkylgruppen oder/und $C_1$-$C_4$-Alkylgruppen derivatisiert ist.

6. Komplex nach Anspruch 5, **dadurch gekennzeichnet,** daß das Cyclodextrin durch Hydroxypropylgruppen, Hydroxyethylgruppen oder/und Methylgruppen derivatisiert ist.

7. Verfahren zur Herstellung eines Komplexes nach einem der Ansprüche 1 bis 6, **dadurch gekennzeichnet,** daß man geeignete Mengen eines Cyclodextrins und eines organischen Biocids in einem wäßrigen oder/und organischen Lösungsmittelsystem oder in geschmolzenem Zustand zusammengibt und den entstehenden Komplex aus dem Reaktionsgemisch gewinnt.

8. Verwendung eines Komplexes nach einem der Ansprüche 1 bis 6 zur Konservierung von wäßrigen Lösungen.

**9.** Verwendung nach Anspruch 8,
**dadurch gekennzeichnet,** daß die wäßrige Lösung Proteine enthält.

**10.** Verwendung nach Anspruch 9,
**dadurch gekennzeichnet,** daß die Lösung enzymatisch aktive Proteine oder/und immunologisch aktive Proteine enthält.

**11.** Verwendung nach einem der Ansprüche 8 bis 10,
**dadurch gekennzeichnet,** daß die wäßrige Lösung eine diagnostische Testlösung ist.

**12.** Diagnostische Testlösung,
**dadurch gekennzeichnet,** daß sie mindestens einen Komplex nach einem der Ansprüche 1 bis 6 enthält.

**13.** Diagnostische Testlösung nach Anspruch 12,
**dadurch gekennzeichnet,** daß sie den Komplex in einer Konzentration von 0,1 µg/ml bis 50 mg/ml enthält.

**14.** Konservierter diagnostischer Testkit, umfassend Testreagenzien und mindestens einen Komplex nach einem der Ansprüche 1 bis 6.

Europäisches Patentamt

# EUROPÄISCHER RECHERCHENBERICHT

Nummer der Anmeldung
EP 94 10 6344

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| X<br><br><br><br><br><br>Y | WO-A-86 03939 (CHINOIN GYOGYSZER ES VEGYESZETI TERMEKEK GYARA RT)<br>* Seite 3, Zeile 22 *<br>* Seite 4, Zeile 7 *<br>* Seite 4, Zeile 17 - Zeile 22 *<br>* Ansprüche 1,6 *<br>--- | 1,7<br><br><br><br><br><br>8-14 | C08B37/16<br>A01N25/10<br>G01N33/50 |
| Y<br><br>D | EP-A-0 467 337 (BOEHRINGER MANNHEIM GMBH)<br>* Ansprüche *<br>& DE-A-40 22 878 (BOEHRINGER MANNHEIM GMBH)<br>--- | 8-14 | |
| Y | DATABASE WPI<br>Week 8430,<br>Derwent Publications Ltd., London, GB;<br>AN 84-185578<br>& JP-A-59 104 556 (SEKISUI CHEMI IND KK) 16. Juni 1984<br>* Zusammenfassung *<br>--- | 8-14 | |
| X | EP-A-0 215 169 (SEUIWA TECHNOLOGICAL LABORATORIES LTD)<br>* Spalte 1, Zeile 32 - Zeile 34 *<br>* Ansprüche *<br>--- | 1,7 | RECHERCHIERTE SACHGEBIETE (Int.Cl.5)<br>C08B<br>A01N<br>G01N |
| X | US-A-4 883 785 (CHOW ET AL.)<br>* Spalte 3, Zeile 26 - Zeile 30 *<br>* Beispiel 1 *<br>--- | 1,7 | |
| X | EP-A-0 186 146 (JAPAN LIQUID CRYSTAL CO.)<br>* Spalte 1, Zeile 5 - Zeile 11 *<br>* Seite 4, Zeile 20 - Zeile 30 *<br>* Beispiel 23 *<br>---<br>-/-- | 1,2,7 | |

Der vorliegende Recherchenbericht wurde für alle Patentansprüche erstellt

| Recherchenort | Abschlußdatum der Recherche | Prüfer |
|---|---|---|
| DEN HAAG | 19. Juli 1994 | Mazet, J-F |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)

Europäisches Patentamt

# EUROPÄISCHER RECHERCHENBERICHT

Nummer der Anmeldung
EP 94 10 6344

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| A | EP-A-0 306 455 (WARNER-LAMBERT COMPANY)<br>* Spalte 6, Zeile 5 - Zeile 52 *<br>* Spalte 7, Zeile 1 - Zeile 13 *<br>* Ansprüche 1,2,7 *<br>--- | 1,7 | |
| A | US-A-4 774 329 (FRIEDMAN)<br>* Spalte 3, Zeile 23 - Zeile 38 *<br>* Ansprüche *<br>--- | 4-7 | |
| A | DATABASE WPI<br>Week 8338,<br>Derwent Publications Ltd., London, GB;<br>AN 83-767169<br>& JP-A-58 134 004 (KYUSHU SANKYO KK) 10. August 1983<br>* Zusammenfassung *<br>----- | | |

RECHERCHIERTE SACHGEBIETE (Int.Cl.5)

Der vorliegende Recherchenbericht wurde für alle Patentansprüche erstellt

| Recherchenort | Abschlußdatum der Recherche | Prüfer |
|---|---|---|
| DEN HAAG | 19. Juli 1994 | Mazet, J-F |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPO FORM 1503 03.82 (P04C03)